# EUROPEAN PATENT APPLICATION

(11) **EP 2 455 120 A1**
(43) Date of publication of application: **23.05.2012**
(21) Application number: 10191801.9
(22) Date of filing: 19.11.2010
(51) Int. Cl.: A61M 5/178, H02K 7/18, H02K 35/00

(54) **Medical device comprising mechanical-electrical transducing means**

(71) Applicant: Sanofi-Aventis Deutschland GmbH, 65929 Frankfurt (DE)
(72) Inventor: Pommereau, Christian, 65926 Frankfurt am Main (DE)

(57) **Abstract**

Medical device comprising mechanical-electrical transducing means

The present invention relates to a medical device for administering a dose of a medicament to a user, comprising:
- at least one housing component (12),
- a drive mechanism (18) to be operably engaged with a cartridge being filled with the medicament,
- at least one electricity-consuming member (24, 26) and
- an mechanical-electrical transducing means (14) adapted to transduce mechanical energy into electrical energy provided to the electricity-consuming member (24, 26).

## Description

### Field of the invention

The present invention relates to a medical device and in particular to medical devices adapted for administering a dose of a medicament to a user and further having at least one electricity-consuming member, e.g. for driving the medical device and/or for driving associated auxiliary device components. Generally, the invention relates to a large variety of different medical devices adapted for various purposes. Among those, the invention particularly focuses on drug delivery devices of e.g. pen-injector type for administering a dose of a liquid medicament by way of injection, without being limited thereto.

### Background and Prior Art

Medicinal products and medicaments have to be administered in various different ways. For instance, parenteral pharmaceuticals are typically administered by way of injection, whereas other pharmaceuticals forms require e.g. deglutition and/or inhaling.

Especially such patients suffering chronic diseases have to take medicaments according to a given time schedule on a regular basis, like two, three or four times a day. Many of such patients are already accustomed to administer the medicament by themselves, in a home environment. For instance, patients suffering diabetes make commonly use of a drug delivery device in form of a pen-type injector having a cartridge placed therein being pre-filled with a particular medicament, like insulin. Such drug delivery devices are rather small in size and weight. They are transportable and the patient may conduct a dose injection procedure almost anywhere at any time.

Even though many of these devices operate all mechanically, a growing number of medical devices come along with a multitude of functions being electrically implemented.

Medical devices may for instance comprise liquid crystal displays or may have electrically implemented control mechanism. However, it remains a problem with such electrically based or electricity-consuming functions of a portable medical to provide a persistent power supply.

The use of batteries is generally not particularly environment-friendly. Also with batteries, even rechargeable batteries or accumulators, the user may not have an appropriate battery or battery charger at hand when needed.

### Objects of the invention

It is therefore an aim of the present invention to provide a medical device having at least one electricity-consuming member and being further provided with an efficient, environment-friendly and rather flexible as well as sustainable and persistent power supply. Moreover, the medical device should be light-weight and cost-efficient in production.

### Summary of the Invention

The medical device according to the present invention is adapted for administering a dose of a medicament to a user. The medical device at least comprises one housing component and a drive mechanism to be operably engaged with a cartridge being pre-filled with the medicament to be administered. Moreover, the medical device comprises at least one electricity consuming member and further has a mechanical-electrical transducing means being adapted to transduce mechanical energy into electrical energy that is to be provided to and consumed by the electricity-consuming member.

Hence, the medical device is equipped with a mechanical-electrical transducing means allowing to generate electric energy on demand simply by a user-driven actuation. The mechanical-electrical transducing means of the medical device therefore provide user-driven and user-generated electrical power to the device and its electricity-consuming members or components.

It is of advantage, that the mechanical-electrical transducing means are muscle operable, so that a user becomes enabled to generate required electric power on demand. Hence, means for storing electric energy can be reduced to a minimum or can in principle even be entirely eliminated. The user-driven mechanical-electrical transducer allows to provide the electricity-consuming member of the medical device with sufficient electrical energy independent of a state of charge of an optional battery.

In a preferred embodiment, the mechanical-electrical transducing means comprise a dynamo being operably engaged with an actuating member to be driven and actuated by a user of the device. Preferably, the actuating member is to be hand-driven by a user, e.g. by pulling and/or pushing the actuating member, which for instance, can be designed as a pivotable lever attached to the housing component of the medical device. Typically, the actuating member can be actuated against the action of a restoring force, which is e.g. provided by a spring element. This way, the actuating member can be displaced or pivoted back and forth, wherein at least one direction of movement is mechanically coupled to an electricity generating member of the dynamo.

In another preferred embodiment, the mechanical-electrical transducing means are integrated into the at least one housing component. Hence, the dynamo or mechanical-electrical transducer is entirely integrated into the medical device. Consequently, the medical device is inherently equipped with a mechanical-electrical user-driven power source. Assuming the proper operation of the transducer, the medical device in principle cannot run out of electric power.

In an alternative embodiment, the mechanical-electrical transducing means are detachably connected to the housing component or other components of the medical device. Here, it is also conceivable, that the mechanical-electrical transducing means are designed as a separate component to be only frequently electrically connected with the medical device, e.g. for re-charging of a rechargeable battery.

In preferred embodiments, the mechanical-electrical transducer is designed and implemented as an integral component of the medical device and/or of its housing component.

According to another embodiment, the actuating member is pivot-mounted in or at the housing component of the medical device. Preferably, the actuating member can be pivoted from an outer, radially protruding idle position to an inner end position under the effect of an inwardly directed force to be provided by a user of the device. For not hindering functionality and handling of the medical device, the actuating member may be secured at the housing component preferably in its end position, e.g. when there is no need for generating electric energy.

If the medical device for instance comprises a cylindrical shape, it is of particular benefit, when the actuating member protrudes from the circumferential sidewall of the housing component, whereas other driving or control means of the medical device are arranged at a distal and/or proximal end of the cylindrical housing. The actuating member should distinguish in size and geometry from other controlling components of the medical device in order to prevent unintended misuse of the device.

For this purpose, the housing component may comprise a corresponding receptacle adapted to receive the actuating member preferably aligned with the outer circumference of the housing component when transferred into its end position. Here, the actuating member may be locked in its end position by some kind of mechanical interlock means. For generating electric power on demand, said interlock means can for instance be released by the user and as a consequence, the actuating member may return into its idle position, in which it is ready to become repeatedly depressed and actuated by the user.

Furthermore and according to another embodiment, the housing component is of substantially cylindrical and/or elongated geometry. Here, the actuating member is displaceably mounted to the housing component in radial direction. Moreover, the actuating member may be alternately translationally and/or pivotally displaceable between an initial and an end position either by a pivoting or a translational displacement, preferably in radial direction with respect to the geometry of the housing component.

In still another embodiment, the medical device further comprises at least one electrical energy storage device, such like a re-chargeable battery or capacitor. Said energy storage device is electrically connected to the at least one electricity-consuming member and to the mechanical-electrical transducing means. The electrical energy storage device may serve as a buffer for at least temporally storing electrical energy generated by the transducing means. Since the intensity of mechanical actuation of the mechanical-electrical transducer may be subject to fluctuations, by way of the energy storage device, such temporal fluctuations of generated electrical power can in principle be levelled out.

According to a number of further embodiments, the electricity-consuming member may comprise a large variety of different devices and/or systems to be implemented into the medical device. For instance, the electricity consuming member may comprise an electronic display indicating information about the filling level of the cartridge and/or about details related to the process of administering the medicament. Additionally, the medical device may be equipped with an electronic warning system visually or acoustically indicating to a user, that an error or some kind of malfunction has occurred or is likely to occur in the near future.

For instance, the electricity-consuming member may be designed as a voice control system and may therefore comprise a microphone and a loudspeaker in order to phonetically interact with the user. Such a control system is of particular benefit for users suffering impaired vision who would otherwise not be able to handle the medical device appropriately.

In still another aspect, the electricity-consuming member may comprise lightning means for at least illuminating parts of the device, e.g. a display and/or for illuminating a user's area of treatment. This may even allow to make use of the medical device in a rather dark environment. Preferably, the lighting means comprise one or several light emitting diodes.

In further embodiments, it is conceivable that the electricity-consuming member is even part of the drive mechanism of the medical device. The electricity-consuming member may comprise an electrical drive or electrical servo drive being operably engaged with the drive mechanism of the medical device, e.g. for expelling a dose of the medicament from the cartridge. Since the energy supply may be persistently coupled to the medical device, even such functions concerning patient safety can in principle be electrically implemented.

However, when even basic functions of the medical device require electric energy and hence electric power, the device should be additionally provided with precaution means adapted to ensure that prior to execution of an electricity-consuming function, sufficient electric power is presently available.

In still another, alternative or additional embodiment, the electricity-consuming member comprises electric authentication means, such like RFID reading components and/or RFID tags. This way, an electrically implemented authentication scheme can be provided and counterfeited medicament cartridges can be automatically identified by the medical device. In response of detecting a counterfeited or falsified cartridge, the medical device is blocked or at least some of its functions are become deactivated.

Additionally, or alternatively, the electricity-consuming member may comprise electric communication means and/or at least an electric based communication port, allowing to transfer data between the medical device and a computer, a personal digital assistant or other electronic consumer products. This way, a momentary status of the medical device as well as a history of repeated usage of the device can be stored, preferably by way of a non-volatile memory of the medical device. This user- or use-related data acquired during repeated usage of the medical device can then be transferred to a computer and can be further analysed by medicinal staff, e.g. in order to survey and/or to analyze administering of the medicament on a long term time scale.

Typically, such electronic-based communication ports may for instance comprise standard hardware components and transmission protocols, like USB interfaces or even wireless interfaces, e.g. on the basis of RF-technology.

Furthermore, the electricity-consuming member may additionally or alternatively comprise detection means being adapted to recognize whether the mechanical-electrical transducing means is operably connected to the housing component. Additionally or alternatively, the detection means may be adapted to identify, whether the mechanical-electrical transducer is in a state of operation. Whenever the detection means identify that the transducer is inoperable, at least some functions of the medical device are deactivated and are at least temporally inaccessible. Otherwise, there might be a danger that electric power consuming functions of the medical device are triggered and initialized without having enough electrical power for executing and terminating said function in an appropriate way.

According to another preferred embodiment of the invention, the medical device further comprises a mechanical interlock adapted to detect the operability of the mechanical-electrical transducing means. The mechanical interlock is further adapted to mechanically block the drive mechanism of the medical device if the transducing means were identified as being inoperable. This way, an all-mechanical securing mechanism can be implemented which is adapted to inhibit device operation if the electrical power generating transducer is not ready to use.

Generally, the mechanical-electrical transducing means can be universally adapted and designed for a large variety of portable or even immobile medical devices.

In a further preferred embodiment, the medical device comprises a drug delivery device and in particular a syringe and/or a pen-type injecting device being adapted to administer a pre-defined dose of a medicament by way of injection. The drug delivery device may comprise multiple housing components, such like a body of a housing being adapted to receive a drive mechanism to be operably engaged with a pre-filled cartridge. In another housing component, typically in a cartridge holder, a single-use or replaceable cartridge can be arranged.

Typically, a distal end section of the cartridge holder is adapted to receive a piercing or needle assembly, typically a double-tipped injection needle, which on the one hand is adapted to penetrate a distally located seal of the cartridge, and which on the other hand is intended to pierce biological tissue for hypodermically injecting the medicament contained in the cartridge. The cartridge, typically comprising a cylindrical barrel comprises a piston slidably disposed therein acting as a proximal seal to be displaced in distal direction for expelling a pre-defined dose of the medicament.

In a typical mode of operation, a piston rod, to be axially driven by a drive mechanism of the drug delivery device is operably connected or operably engaged with the piston of the cartridge in order to displace said piston in distal direction, hence, in a dose injecting way in a controlled manner.

In another preferred embodiment, the drug delivery device, in particular, the pen-type injector comprises a cartridge filled with the medicament. The drug delivery device of e.g. pen-injector type may be designed as disposable or re-usable device, wherein upon consumption of the medicament either the entire device is to be discarded or the cartridge is to be replaced by a new one, respectively.

Furthermore and according to another aspect, the medical device comprises an inhaler device adapted to provide the medicament in form of an aerosol to be inhaled by the patient.

The term "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, a antibody, an enzyme, an antibody, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39); or

des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39),
wherein the group -Lys6-NH2 may be bound to the C-terminus of the Exendin-4 derivative;
or an Exendin-4 derivative of the sequence
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

It will be further apparent to those skilled in the pertinent art that various modifications and variations can be made to the present invention without departing from the spirit and scope of the invention. Further, it is to be noted, that any reference signs used in the appended claims are not to be construed as limiting the scope of the present invention.

### Brief Description of the Drawings

In the following, preferred embodiment of the invention will be described in detail by making use of the embodiments illustrated in the figures, in which:
- Figure 1: schematically illustrates a drug delivery device comprising a mechanical-electrical transducing means and
- Figure 2: shows a perspective illustration of a drug delivery device having two electricity-consuming members.

### Detailed Description

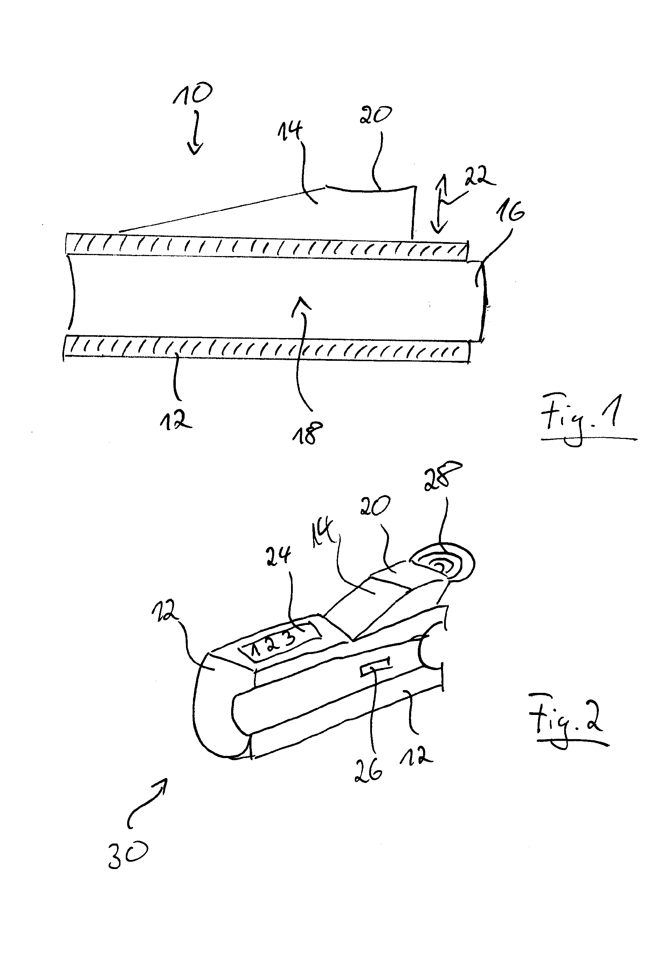

The cross sectional illustration according to Figure 1 exemplary shows a housing component 12 of a drug delivery device, e.g. a proximal component of a pen-type injector. The housing component 12 is of substantially cylindrical geometry and serves to house a drive mechanism 18 adapted to axially displace a piston rod of the drive mechanism in distal direction, which is not particularly illustrated here. For setting and/or dispensing of a dose, the device 10 comprises a proximally located dose button, which can for instance be rotated for setting of a dose and which can be pushed or pulled for starting a dose dispensing procedure.

Additionally, the drug delivery device 10 according to Figure 1 comprises a mechanical-electrical transducing means, e.g. implemented as a dynamo being not further illustrated here. The dynamo is operably engaged with an actuating member 14 radially protruding from the outer circumference of the housing component 12. As indicated by the arrow 22 the actuating member 14 can be radially displaced or pivoted in order to drive the dynamo and to or transduce mechanical into electrical energy. As further illustrated in Figure 1 the actuating member 14 comprises a recessed grip or finger grip 20 adapted to facilitate repeated depressing of the actuating member 14.

In Figure 2 another drug delivery device 30 is illustrated also having a housing component 12 of substantially cylindrical geometry and also having a pivot mounted or translationally displaceable actuating member 14 as already indicated in Figure 1. Here, the actuating member 14 at its distal and upper end further comprises another shell-like recessed grip 28 being designed and adapted to receive a thumb of a user. Additionally, in the illustration according to Figure 2 the drug delivery device 30 comprises two different electricity-consuming members 24, 26. Here, at the outer circumference of the housing 12 a display 24 is arranged adapted to indicate and to illustrate numbers, symbols and/or words in order to facilitate operation of the device.

The drug delivery device 30 further has a communication port 26, being e.g. designed as USB-port for connecting the drug delivery device 30 to some kind of analysing instrument, like a personal computer.

### List of Reference Numerals

- 10: drug delivery device
- 12: housing component
- 14: actuating member
- 16: dose button
- 18: drive mechanism
- 20: recessed grip
- 22: actuating direction
- 24: display
- 26: USB-port
- 28: recessed grip
- 30: drug delivery device

## Claims

1. Medical device for administering a dose of a medicament to a user, comprising:
- at least one housing component (12),
- a drive mechanism (18) to be operably engaged with a cartridge being filled with the medicament,
- at least one electricity-consuming member (24, 26) and
- mechanical-electrical transducing means (14) adapted to transduce mechanical energy into electrical energy provided to the electricity-consuming member (24, 26).

2. Medical device according to claim 1, wherein the mechanical-electrical transducing means comprise a dynamo operably engaged with an actuating member (14) to be driven by a user of the device.

3. Medical device according to any one of the preceding claims, wherein the mechanical-electrical transducing means (14) are integrated into the at least one housing component (12).

4. Medical device according to any one of the preceding claims 1 or 2, wherein the mechanical-electrical transducing means are detachably connected to the housing component (12).

5. Medical device according to any one of the preceding claims 2 to 4, wherein the actuating member (14) in an idle position at least partially protrudes from the housing component (12).

6. Medical device according to any one of the preceding claims 2 to 4, wherein the actuating member (14) is pivot-mounted in or at the housing component (12).

7. Medical device according to any one of the preceding claims, wherein the housing component (12) is of substantially cylindrical and/or elongated geometry and wherein the actuating member (14) is displaceably mounted to the housing component (12) in radial direction.

8. Medical device according to any one of the preceding claims, further comprising an electrical energy storage device electrically connected to the at least one electricity-consuming member (24, 26) and to the mechanical-electrical transducing means.

9. Medical device according to any one of the preceding claims, wherein the electricity-consuming member comprises (24, 26):
- an electronic display (24) and/or an electronic warning system, and/or
- a voice control system, and/or
- lightning means for illuminating at least parts of the device and/or for illuminating a user's area of treatment, and/or
- an electrical drive or electrical servo drive, operably engaged with the drive mechanism (18), and/or
- electric authentication means, and/or
- electric communication means and/or at least one electric based communication port (26), and/or
- detection means adapted to recognize whether the mechanical-electrical transducing means (14) is operably connected to the housing component (12).

10. Medical device according to any one of the preceding claims, further comprising a mechanical interlock adapted to detect operability of the mechanical-electrical transducing means (14) and being further adapted to mechanically block the drive mechanism (18) if the transducing means (14) are inoperable.

11. Medical device according to any one of the preceding claims, comprising a drug delivery device and/or syringe and/or a pen-type injecting device designed for administering a predefined dose of the medicament by way of injection.

12. The drug delivery device according to any one of the preceding claims 1 to 10, comprising an inhaler device.

13. The drug delivery device according to any one of the preceding claims, further comprising a cartridge filled with the medicament.
